# FASCICULE DE BREVET EUROPEEN

(11) **EP 1 519 951 B1**
(45) Date de publication et mention de la délivrance du brevet: **23.05.2007**
(21) Numéro de dépôt: 03762746.0
(22) Date de dépôt: 08.07.2003
(51) Int. Cl.: A61K 38/17, A01H 5/00

(54) **PEPTIDES LINEAIRES CATIONIQUES AYANT DES PROPRIETES ANTIFONGIQUES**
LINEARE KATIONISCHE PEPTIDE MIT FUNGIZIDER WIRKSAMKEIT
CATIONIC LINEAR PEPTIDES HAVING ANTIFUNGAL PROPERTIES

(30) Priorité: 08.07.2002 FR 0208565
(43) Date de publication de la demande: 06.04.2005
(73) Titulaire: Diatos S.A., 75014 Paris (FR)
(72) Inventeur: ARRANZ, Valérie, 77122 Monthyon (FR)
(74) Mandataire: Breese, Pierre
(86) Numéro de dépôt international: PCT/FR2003/002123
(87) Numéro de publication internationale: WO 2004/005339

(56) Documents cités:
- WO-A-01/15511
- WO-A-01/64738
- JAVADPOUR MM ET AL.: "De Novo Antimicrobial Peptides with Low Mammalian Cell Toxicity" JOURNAL OF MEDICINAL CHEMISTRY, vol. 39, 1996, pages 3107-3113, XP002231077 cité dans la demande
- DATABASE NCBI [en ligne] Hypothetical protein T05H4.3, 1998 Database accession no. AAB66021 XP002231078

### LISTAGE DE SEQUENCES

<110> DIATOS SA
<120> Peptides linéaires et cationiques ayant des propriétés antibactériennes et/ou anti-fongiques
<130> 25998FR
<140> PCT/FR03/XXXXX
   <141> 2003-07-08
<150> FR02/08565
   <151> 2002-07-08
<160> 10
   <170> PatentIn version 3.1
<210> 1
   <211> 7
   <212> PRT
   <213> unidentified
<220>
   <221> PEPTIDE
   <222> (1) .. (7)
   <223>
<400> 1
   Val Lys Arg Gly Leu Lys Leu
   1 5
<210> 2
   <211> 12
   <212> PRT
   <213> unidentified
<220>
   <221> PEPTIDE
   <222> (1)..(12)
   <223>
<400> 2
   Lys His Leu Lys Lys His Leu Lys Lys His Leu Lys
   1 5 10
<210> 3
   <211> 15
   <212> PRT
   <213> unidentified
<220>
   <221> PEPTIDE
   <222> (1).. (15)
   <223>
<400> 3
   Gly Lys Arg Lys Lys Lys Gly Lys Leu Gly Lys Lys Arg Asp Pro
   1 5 10 15
<210> 4
   <211> 14
   <212> PRT
   <213> unidentified
<220>
   <221> PEPTIDE
   <222> (1)..(14)
   <223>
<400> 4
   Lys Leu Ala Lys Leu Ala Lys Lys Leu Ala Lys Leu Ala Lys
   1 5 10
<210> 5
   <211> 63
   <212> DNA
   <213> unidentified
<220>
   <221> CDS
   <222> (1)..(63)
   <223> Séquence codant le peptide 1
<400> 5
   gtt aaa cgt ggt ttg aaa ttg aaa ttg gct aaa ttg gct aaa aaa ttg      48
   Val Lys Arg Gly Leu Lys Leu Lys Leu Ala Lys Leu Ala Lys Lys Leu
   1               5                   10                  15
   gct aaa ttg gct aaa                                                   63
   Ala Lys Leu Ala Lys
               20
<210> 6
   <211> 21
   <212> PRT
   <213> unidentified
<400> 6
   Val Lys Arg Gly Leu Lys Leu Lys Leu Ala Lys Leu Ala Lys Lys Leu
   1               5                   10                  15
   Ala Lys Leu Ala Lys
               20
<210> 7
   <211> 78
   <212> DNA
   <213> unidentified
<220>
   <221> CDS
   <222> (1)..(78)
   <223> Séquence codant le peptide 2
<400> 7
   aaa ttg gct aaa ttg gct aaa aaa ttg gct aaa ttg gct aaa aaa cat      48
   Lys Leu Ala Lys Leu Ala Lys Lys Leu Ala Lys Leu Ala Lys Lys His
   1               5                   10                  15
   ttg aaa aaa cat ttg aaa aaa cat ttg aaa                              78
   Leu Lys Lys His Leu Lys Lys His Leu Lys
               20                  25
<210> 8
   <211> 26
   <212> PRT
   <213> unidentified
<400> 8
   Lys Leu Ala Lys Leu Ala Lys Lys Leu Ala Lys Leu Ala Lys Lys His
   1               5                   10                  15
   Leu Lys Lys His Leu Lys Lys His Leu Lys
               20                  25
<210> 9
   <211> 87
   <212> DNA
   <213> unidentified
<220>
   <221> CDS
   <222> (1)..(87)
   <223> Séquence codant le peptide 3
<400> 9
   aaa ttg gct aaa ttg gct aaa aaa ttg gct aaa ttg gct aaa ggt aaa      48
   Lys Leu Ala Lys Leu Ala Lys Lys Leu Ala Lys Leu Ala Lys Gly Lys
   1               5                   10                  15
   cgt aaa aaa aaa ggt aaa ttg ggt aaa aaa cgt gat cct                  87
   Arg Lys Lys Lys Gly Lys Leu Gly Lys Lys Arg Asp Pro
               20                  25
<210> 10
   <211> 29
   <212> PRT
   <213> unidentified
<400> 10
   Lys Leu Ala Lys Leu Ala Lys Lys Leu Ala Lys Leu Ala Lys Gly Lys
   1               5                   10                  15
   Arg Lys Lys Lys Gly Lys Leu Gly Lys Lys Arg Asp Pro
               20                  25

## Description

La présente invention concerne le domaine du traitement des infections fongiques chez l'homme, l'animal ou les plantes. Elle vise plus particulièrement l'utilisation de peptides pour la fabrication de compositions destinées à prévenir et/ou traiter des infections par des champignons.

De nombreux micro-organismes pathogènes et responsables de sévères infections chez l'homme et l'animal présentent des résistances aux antibiotiques couramment utilisés en clinique. Le développement croissant des résistances microbiennes est un problème majeur de santé publique. Aussi, depuis quelques années, un intérêt considérable s'est développé pour l'identification de nouvelles molécules et particulièrement pour l'étude de peptides courts (15 à 30 acides aminés) possédant des activités antibactériennes et antifongiques et ayant un mode d'action différent des antibiotiques usuellement utilisés. De nombreux peptides dérivent de protéines humaines, animales et végétales connues comme possédant des propriétés antimicrobiennes tandis que d'autres sont générés de façon synthétique sur la base d'études physico-chimiques.

Le peptide 14 mer linéaire de séquence KLAKLAKKLAKLAK a été décrit dans la littérature comme un peptide non hémolytique ayant une activité antibactérienne (Javadpour MM, Juban MM, Lo WC, Bishop SM, Alberty JB, Cowell SM, Becker CL, McLaughlin ML. De novo antimicrobial peptides with low mammalian cell toxicity. J Med Chem. 1996 Aug 2;39(16):3107-23). Les études restreintes menées avec ce peptide ont montré une activité sur trois souches de bactéries (E. coli, P. aeruginosa et S. aureus) pendant un temps d'incubation court de 4 heures. Des résultats avec des temps d'incubation plus longs n'ont jamais été décrits.

La Demanderesse a réalisé des études *in vitro* des propriétés antifongiques de ce peptide dans les conditions standard d'évaluation (incubation de 18 heures) qui n'ont montré aucune activité biologique aux concentrations décrites dans la littérature. Il a par contre maintenant été mis en évidence, que de façon inattendue, le motif KLAKLAK était capable de conférer des propriétés antifongiques à des peptides comprenant ce motif et une séquence peptidique n'ayant pas de propriété antifongique.

La présente invention a donc pour objet l'utilisation pour la fabrication d'une composition pharmaceutique, destinée à traiter et/ou prévenir une infection fongique chez l'homme ou l'animal d'un peptide, avantageusement linéaire et cationique, comprenant ou consistant en :
- une première séquence peptidique de formule (KLAKLAK), où K est l'acide aminé Lysine, L est l'acide aminé Leucine et A est l'acide aminé Alanine, et
- une seconde séquence peptidique de formule (B), où B est un peptide de 4 à 15 acides aminés chargé positivement à pH neutre, comprenant ou consistant en au moins un motif peptidique de formule βXXβ, où β est un acide aminé basique et X est n'importe quel acide aminé,
et dans lequel, ladite première séquence peptidique est répétée n fois et ladite seconde séquence peptidique est répétée m fois, n et m étant des nombres entiers compris entre 1 et 5. n est de préférence un nombre entier compris entre 1 et 3. Avantageusement m est supérieur à 1 et les secondes séquences peptidiques (B) sont identiques ou différentes.

Les peptides selon l'invention peuvent donc comprendre plusieurs secondes séquences peptidiques (B) identiques ou différentes et donc plusieurs motifs peptidiques de formule βXXβ, identiques ou différents, dans chacune des secondes séquences peptidiques (B).

Les peptides selon l'invention sont avantageusement linéaires. Ils répondent donc à la formule générale (I) suivante : (KLAKLAK)ₙ(B)ₘ, dans laquelle les première(s) et seconde(s) séquences peptidiques sont :
- alternées, ou
- regroupées à l'une et/ou l'autre des extrémités N et C terminales d'une ou desdites première(s) ou seconde(s) séquences peptidiques.

De préférence, dans les peptides de l'invention, la ou les premières séquences peptidiques sont regroupées à l'une et/ou l'autre des extrémités N et C terminales de la ou des seconde(s) séquence(s) peptidique(s).

Préférentiellement, lesdites première(s) et seconde(s) séquences peptidiques sont liées par des liaisons covalentes, avantageusement peptidiques.

Dans le motif peptidique de formule βXXβ, β est un acide aminé basique de préférence choisi dans le groupe comprenant l'arginine (R) et la lysine (K) et X est de préférence choisi dans le groupe comprenant la leucine (L), la glycine (G) et l'histidine (H).

A titre d'exemple spécifique de la seconde séquence peptidique entrant dans la composition des peptides selon l'invention, on peut citer les séquences suivantes :
- SEQ ID NO.1 : VKRGLKL
- SEQ ID NO.2 : KHLKKHLKKHLK
- SEQ ID NO.3 : GKRKKKGKLGKKRDP

Ces secondes séquences peptidiques peuvent être associées à une première séquence peptidique répétée deux fois : KLAKLAKKLAKLAK (SEQ ID NO.4) de façon à générer des peptides linéaires cationiques ayant une activité antifongique après 18 heures d'incubation avec les champignons filamenteux ou les levures.

Les peptides selon l'invention peuvent être préparés par synthèse chimique ou par génie génétique dans une cellule procaryote comme une bactérie, dans une cellule eucaryote comme une levure, une cellule CHO, une cellule NSO, chez un animal transgénique, par exemple dans le lait de lapin, de chèvre, de brebis, transgéniques ou dans une plante transgénique comme, par exemple, dans des plants de tabac.

L'invention concerne aussi des équivalents fonctionnels des peptides définis ci-dessus, tels que des peptides comprenant des modifications issues de processus post-traductionnels comme la glycosylation ou de modifications chimiques telles que le couplage avec des lipides, sucres, séquences de nucléotides dès lors que ces modifications ne modifient pas l'activité antifongique desdits peptides conformément aux tests donnés dans la partie expérimentale ci-après. Les équivalents fonctionnels comprennent aussi des peptides dont un ou plusieurs acides aminés sont des acides aminés de conformation D. L'invention couvre également les rétro-peptides et les rétro-inverso-peptides.

La présente invention décrit donc une composition pharmaceutique, cosmétique ou agroalimentaire comprenant comme principe actif au moins un peptide tel que défini précédemment associé dans ladite composition avec un ou plusieurs véhicules, diluants ou excipients pharmaceutiquement acceptables. Les peptides selon l'invention présentent une faible toxicité et de plus, ces peptides sont non hémolytiques.

Les véhicules, diluants et excipients sont choisis en fonction du type d'application de la composition à titre pharmaceutique, cosmétique ou agronomique.

Ainsi, l'invention a encore pour objet l'utilisation d'un peptide tel que défini précédemment pour la préparation d'une composition pharmaceutique, cosmétique, dermatologique ou agroalimentaire à visée antifongique.

Les peptides de l'invention possèdent une activité contre une large variété de champignons filamenteux et levures alors que les secondes et premières séquences prises isolement n'ont pas d'activité dans les conditions d'évaluation choisies.

Les compositions selon l'invention sont utiles tant à titre préventif que curatif.

L'administration de composition pharmaceutique selon l'invention peut être réalisée par l'un quelconque des modes d'administration acceptés pour les agents thérapeutiques. Ces procédés comprennent l'administration systémique, topique ou encore centrale, par exemple par voie chirurgicale intracrânienne ou encore l'administration intra-oculaire. On peut citer aussi l'implantation sous-cutanée d'implants biodégradable.

L'administration orale peut se faire au moyen de comprimés, gélules, capsules molles, y compris les formulations à libération différée ou prolongée, pilules, poudres, granules, élixirs, teintures, suspensions, sirops et émulsions. Cette forme de présentation est plus particulièrement adaptée au passage de la barrière intestinale et à l'utilisation la plus commune des composés antifongiques.

L'administration parentérale de composés antifongiques se fait généralement par injection intramusculaire ou intraveineuse par perfusion. Les compositions injectables peuvent être préparées sous des formes classiques, soit en suspension ou solution liquide soit sous forme solide convenant à une dissolution extemporanée dans un liquide adéquat, y compris les formulations à libération différée ou prolongée comme l'inclusion des peptides dans des micro ou nano particules biodégradables de formulation lipidique ou de formulation de dextran ou encore de PLGA ou équivalents. Cette forme de présentation est plus particulièrement adaptée au passage de la barrière hémato-encéphalique et à l'utilisation hospitalière des composés antifongiques.

Une possibilité pour l'administration parentérale utilise l'implantation d'un système à libération lente ou libération prolongée qui assure le maintien d'un niveau constant de dose.

Une autre possibilité consiste à fixer par adsorption ou autre les peptides de l'invention sur un support, comme un cathéter, une prothèse ou une colle biologique.

Pour l'administration intranasale, on peut utiliser des véhicules intranasaux convenables.

D'autres préparations topiques habituelles comprennent les crèmes, les onguents, les lotions, les gels et les sprays aérosols. Ces derniers sont plus particulièrement adaptés pour le traitement des infections broncho-pulmonaires fongiques.

L'utilisation des peptides de l'invention dans des compositions cosmétiques est utile à titre essentiellement préventif consistant à inclure lesdits peptides dans les crèmes, les vernis à ongle, les produits d'hygiène des organes génitaux, les pâtes de dentifrices, les solutions d'hygiène buccales ou à les inclure dans des micro-particules à diffusion lente, en phase aqueuse, incluses par exemple dans des couches-culottes, des cotons-tiges, des pansements, des cotons à démaquiller, des serviettes hygiéniques ou encore des litières pour animaux.

En fonction du mode d'administration, les composés peuvent être sous forme solide, semi-solide ou liquide.

Pour les compositions solides, tels que comprimés, pilules, poudres ou granulés à l'état libre ou inclus dans des gélules, le principe actif peut être combiné avec :
- des diluants, par exemple le lactose, le dextrose, le sucrose, le mannitol, le sorbitol, la cellulose et/ou la glycine ;
- des lubrifiants, par exemple la silice, le talc, l'acide stéarique, son sel de magnésium ou de calcium et/ou le polyéthylèneglycol ;
- des liants, par exemple le silicate de magnésium et d'aluminium, la pâte d'amidon, la gélatine, la gomme adragante, la méthylcellulose, la carboxyméthylcellulose sodique et/ou la polyvinylpyrrolidone ; le cas échéant,
- des désintégrants, par exemple l'amidon, l'agar, l'acide alginique ou son sel de sodium, ou des mélanges effervescents ; et/ou
- des absorbants, colorants, aromatisants et édulcorants. Les excipients peuvent être par exemple du mannitol, lactose, amidon, stéarate de magnésium, saccharine sodique, talc, cellulose, glucose, sucrose, carbonate de magnésium et analogues de qualité pharmaceutique.

Pour les compositions semi-solides, telles que suppositoires, l'excipient peut, par exemple, être une émulsion ou suspension grasse, ou à base de polyalkylèneglycol, tel que le polypropylène-glycol.

Les compositions liquides, en particulier injectables ou à inclure dans une capsule molle, peuvent être préparées par exemple par dissolution, dispersion, etc. du principe actif dans un solvant pharmaceutiquement pur tel que, par exemple, l'eau, le sérum physiologique, le dextrose aqueux, le glycérol, l'éthanol, une huile et analogues.

Les peptides selon l'invention peuvent également être administrés sous la forme de systèmes de libération du type liposomes, tels que sous la forme de petites vésicules unilamellaires, de grandes vésicules unilamellaires et de vésicules multilamellaires. Les liposomes peuvent être formés à partir d'une diversité de phospholipides, contenant du cholestérol, de la stéarylamine ou des phosphatidylcholines. Dans une forme d'exécution, un film de composants liquides peut être hydraté avec une solution aqueuse du médicament pour former une couche lipidique encapsulant le médicament.

Les compositions selon l'invention peuvent être stérilisées et/ou contenir des adjuvants et substances auxiliaires non toxiques tels que des agents de conservation, de stabilisation, de mouillage ou d'émulsification, des agents favorisant la dissolution, des sels pour régler la pression osmotique et/ou des tampons. En outre, elles peuvent également contenir d'autres substances présentant un intérêt thérapeutique. Les compositions sont préparées, respectivement, par des procédés classiques de mélange, granulation ou enrobage et elles contiennent d'environ 0,1 à 75%, de préférence d'environ 1 à 50 %, de principe actif.

Les peptides selon l'invention peuvent être également couplés avec des polymères solubles tels que des supports de médicament ciblables. De tels polymères peuvent comprendre la polyvinylpyrrolidone, le copolymère pyrane, le polyhydroxypropyl-méthacrylamide-phénol, le polyhydroxy-éthyl-aspanamide-phénol ou le poly(oxyde d'éthylène)-polylysine substitué par des résidus palmitoyle, le dextran. En outre, les composés selon la présente invention peuvent être couplés à une classe de polymères biodégradables utiles pour réaliser une libération maîtrisée d'un médicament, par exemple, le poly(acide lactique), la poly(epsilon-caprolactone), le poly(acide hydroxybutyrique), les polyorthoesters, les polyacétals, les polydihydropyranes, les polycyanoacrylates et les copolymères d'hydrogel séquencés réticulés ou amphipatiques.

La posologie pour l'administration des peptides selon l'invention est choisie en fonction de nombreux facteurs y compris le type, l'espèce, l'âge, le poids, le sexe et l'état médical du sujet ; la gravité de l'état à traiter ; la voie d'administration ; l'état des fonctions rénale et hépatique du sujet et la nature du composé particulier, ou sel, employé. Un médecin ou vétérinaire normalement expérimenté déterminera facilement, et prescrira, la quantité efficace pour prévenir, contrarier ou stopper le progrès de l'état médical à traiter.

Une composition selon l'invention peut contenir de 0,1 à 99%, de préférence 1 à 70% de principe actif.

A titre d'exemples, les posologies orales des peptides selon l'invention seront comprises entre environ 0,5 et 1 mg/jour par voie orale et, de préférence fournies sous la forme de comprimés contenant 0,5, 1, 2,5, 5, 10, 15, 25, 50, 100, 250, 500 et 1.000 mg de principe actif. Les concentrations plasmatiques efficaces de peptides seront obtenues à partir d'une posologie allant de 0,002 mg à 50 mg par kg de poids corporel et par jour.

Les peptides de l'invention peuvent être administrés sous la forme de doses quotidiennes uniques, ou en deux, trois ou quatre doses par jour.

L'invention envisage non seulement l'administration des peptides décrits précédemment mais également l'utilisation des séquences polynuclucléotidiques codant ces peptides pour transformer cellules végétales. Ces séquences sont utilisées conformément aux techniques du génie génétique décrit dans la littérature.

En conséquence, l'invention décrit également un polynucléotide codant un peptide décrit précédemment, une molécule d'acide nucléique, ADN ou ARN, comme un vecteur, comprenant ledit polynucléotide, et une cellule végétale comprenant ladite molécule d'acide nucléique.

A titre d'exemples de polynucléotides, qui peuvent être utilisés pour transformer des cellules végétales on peut citer :
- la séquence 5' GTT AAA CGT GGT TTG AAA TTG AAA TTG GCT AAA TTG GCT AAA AAA TTG GCT AAA TTG GCT AAA 3' (SEQ ID NO.5) codant le peptide VKRGLKLKLAKLAKKLAKLAK (SEQ ID NO. 6),
- la séquence 5' AAA TTG GCT AAA TTG GCT AAA AAA TTG GCT AAA TTG GCT AAA AAA CAT TTG AAA AAA CAT TTG AAA AAA CAT TTG AAA 3' (SEQ ID NO.7) codant le peptide KLAKLAKKLAKLAKKHLKKHLKKHLK (SEQ ID NO. 8).
- la séquence 5' AAA TTG GCT AAA TTG GCT AAA AAA TTG GCT AAA TTG GCT AAA GGT AAA CGT AAA AAA AAA GGT AAA TTG GGT AAA AAA CGT GAT CCT 3' (SEQ ID NO.9) codant le peptide KLAKLAKKLAKLAKGKRKKKGKLGKKRDP (SEQ ID NO. 10).

L'invention s'intéresse donc aussi aux applications agronomiques des peptides décrits précédemment pour rendre des plantes résistantes aux champignons phyto-pathogènes et réduire ainsi l'utilisation à titre préventif ou curatif de pesticides chimiques toxiques pour l'environnement. L'application directe sur la plante d'une quantité efficace de peptides antifongique ou d'une composition en contenant représente une première forme de mise en oeuvre de l'application agronomique. Une deuxième forme de mise en oeuvre de cette application est basée sur des techniques de transgénèse consistant à incorporer de façon stable dans l'ADN d'une cellule végétale, une séquence polynucléotidique codant pour un ou plusieurs peptides antifongiques ci-dessus. Les cellules végétales ainsi transformées permettent de régénérer une plante transmettant le caractère de résistance aux infections fongiques à sa descendance. A titre d'exemples de plantes, on peut citer le riz, le mais, le colza, la betterave, le blé, le tabac, la tomate, la pomme de terre.

Les acides aminés des séquences peptidiques sont généralement représentés par leur code à une lettre, mais ils peuvent être aussi représentés par leur code à trois lettres selon la nomenclature ci-dessous :

| | | |
|---|---|---|
| A | Ala | Alanine |
| C | Cys | Cystéine |
| D | Asp | Acide Aspartique |
| E | Glu | Acide Glutamique |
| F | Phe | Phénylalanine |
| G | Gly | Glycine |
| H | His | Histidine |
| I | Ile | Isoleucine |
| K | Lys | Lysine |
| L | Leu | Leucine |
| M | Met | Méthionine |
| N | Asn | Asparagine |
| P | Pro | Proline |
| Q | Gln | Glutamine |
| R | Arg | Arginine |
| S | Ser | Sérine |
| T | Thr | Thréonine |
| V | Val | Valine |
| W | Trp | Tryptophane |
| Y | Tyr | Tyrosine |

D'autres avantages et caractéristiques de l'invention apparaîtront des exemples qui suivent, donnés à titre illustratif, et dans lesquels il sera fait référence aux dessins en annexe où :

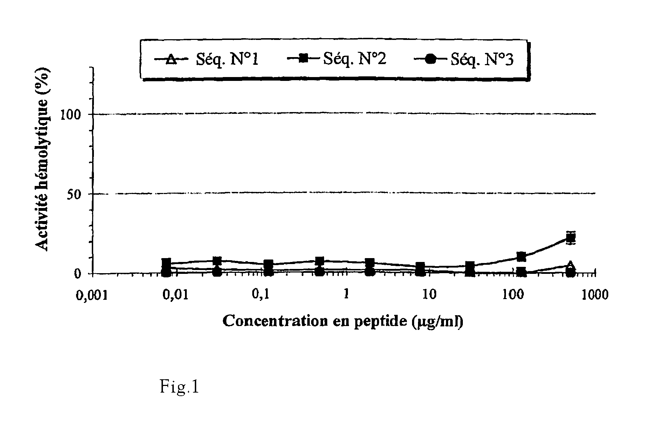

- La figure 1 rapporte l'étude de l'activité hémolytique des peptides No. 1, No. 2 et No. 3 respectivement de séquences SEQ ID NO.6, SEQ ID NO.8, SEQ ID NO.10 sur les érythrocytes humains.

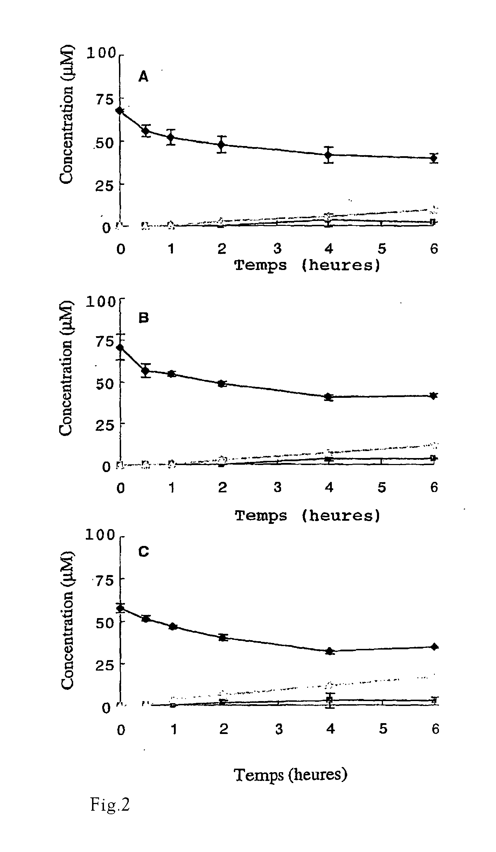

- La figure 2 rapporte la stabilité du peptide No. 2 (SEQ ID NO.8) dans le plasma humain.

### MATERIEL.

### 1) Peptides linéaires.

No. 1 : VKRGLKLKLAKLAKKLAKLAK (SEQ ID NO.6)
No. 2 : KLAKLAKKLAKLAKKHLKKHLKKHLK (SEQ ID NO.8)
No. 3 : KLAKLAKKLAKLAKGKRKKKGKLGKKRDP (SEQ ID NO.10)

### 2) Champignons filamenteux et levures.

Les souches utilisées et référencées dans les divers résultats sont issues des banques de collections de l'ATCC (American Type Culture Collection) et de l'Institut Pasteur.

### 3) Cellules.

Hématies humaines issues de sang total provenant de l'Etablissement Français du Sang (EFS) et conservées à +4°C.

### 4) Préparation des peptides.

Les synthèses peptidiques ont été réalisées selon les techniques connues de l'homme de métier. Les peptides sont solubles dans l'eau.

### RESULTATS.

### I - Détermination de la concentration minimale inhibitrice (CMI) en milieu liquide pour les champignons filamenteux et les levures.

Concentrations en peptide testées : de 0,2µg/ml à 100µg/ml.

Le tableau 4 ci-dessous rapporte l'activité des peptides contre les champignons.

**Tableau 4**

| Séquence CMI (µg/ml) | C. neoformans Cn52D | C. albicans ATCC 90028 | A. Fumigatu s 2001/143.02 |
|---|---|---|---|
| No. 1 | 12,5 | AND* | AND |
| No. 2 | 1,56 | AND | AND |
| No. 3 | 1,56 | AND | AND |

| | | | |
|---|---|---|---|
| * AND : Activité Non Détectée à 100µg/ml | | | |

Les résultats montrent que les 3 peptides testés sont actifs sur Cryptococcus neoformans.

### II - Evaluation de l'activité hémolytique des peptides.

1x10⁶ érythrocytes humains sont incubés pendant 30 minutes avec des concentrations croissantes de peptides (de 0 à 500µg/ml) dans un volume final de 150µl. L'activité hémolytique est évaluée par mesure de l'absorbance (Abs) à la longueur d'onde 414nm (Absorbance de l'hémoglobine) des surnageants cellulaires. L'absorbance du surnageant après incubation des érythrocytes dans l'eau correspond à 100% (Abs₁₀₀) d'hémolyse et l'absorbance du surnageant après incubation des érythrocytes dans le PBS correspond à 0% d'hémolyse (Abs₀). L'activité hémolytique des peptides exprimée en pourcentage correspond à (Abs_{peptide} - Abs₀) / Abs₁₀₀ X 100.

La figure 4 rapporte l'étude de l'activité hémolytique des peptides No. 1, No. 2 et No. 3 sur les érythrocytes humains. Les peptides ne montrent pas d'activité hémolytique.

### III - Evaluation de la stabilité des peptides.

Un volume de 3,9 ml de plasma humain est ajouté à 100 µl de peptide No. 2 à 2 mM. La stabilité est réalisée à 37°C. Après 0, 0.5, 1, 2, 4 et 6 heures, 3x200µl sont prélevés et ajoutés à 10µl de standard interne (peptide No. 1) à 500µM dans de l'eau, auxquels 800 µl d'acide trifluoroacétique (TFA) 0,1% dans de l'eau sont additionnés. Un volume de 1 ml de ce mélange est déposé sur la résine C2(EC) préalablement équilibrée avec 1 ml de TFA 0,1% dans de l'eau, 1 ml de TFA 0.1% dans de l'acétonitrile et 1 ml de TFA 0,1% dans de l'eau. Après deux lavages avec 1 ml de TFA 0,1% dans de l'eau, l'élution du peptide et de ces métabolites se fait par 1 ml de TFA 0,1% dans du méthanol. Celui-ci est ensuite séché sous flux d'air et repris dans 200 µl de TFA 0.1% dans de l'eau. Un volume de 100 µl est analysé par HPLC sur une colonne Luna C18(2), 3 µ, 4.6 x 100mm (Phenomenex) avec du TFA 0,1% dans de l'eau comme solvant A et du TFA 0.1% dans de l'acétonitrile comme solvant B. Un gradient de 15-45% de B en 12 minutes est utilisé pour l'élution avec un débit de 1.2 ml/min et la détection est réalisée à 214 nm.

La figure 5 rapporte l'étude de la stabilité du peptide 2 dans trois lots de plasma humain issus de groupes sanguins différents : A⁺ (A); B⁺ (B) et 0⁺ (C). (◆:peptide No. 2, □ et Δ : métabolites

Deux principaux métabolites sont générés par clivage du peptide No. 2. Le peptide No. 2 est dégradé au maximum à 41% après 6 heures d'incubation dans le plasma humain. Quel que soit le groupe sanguin dont est issu le plasma, il n'y a pas de différence significative dans la dégradation du peptide.

### IV - Evaluation de la toxicité in vivo des peptides.

La dose maximale tolérée (DMT) pour chaque peptide est déterminée sur des souris OF1 femelles à 28 jours après une injection. La DMT est définie comme la dose non létale maximale pouvant être injectée. Les peptides sont administrés par voie intraveineuse en perfusion de 15 minutes environ. Le volume d'injection est de 10 µl/g, le temps de perfusion est de 1,2 ml/heure et l'excipient est une solution de NaCl à 0.8%.

Le tableau 5 ci-dessous rapporte les DMT des peptides No. 1 et No. 2.

**Tableau 5**

| Peptide | DMT |
|---|---|
| Peptide 1 | >23.2 mg/kg |
| Peptide 2 | 13.5 mg/kg |

## Revendications

1. Utilisation pour la fabrication d'une composition pharmaceutique, destinée à traiter et/ou prévenir une infection fongique chez l'homme ou l'animal d'un peptide comprenant ou consistant en :
(i) une première séquence peptidique de formule (KLAKLAK), où K est l'acide aminé Lysine, L est l'acide aminé Leucine et A est l'acide aminé Alanine, et
(ii) une seconde séquence peptidique de formule (B), où B est un peptide de 4 à 15 acides aminés chargé positivement à pH neutre, comprenant ou consistant en au moins un motif peptidique de formule βXXβ, où β est un acide aminé basique et X est n'importe quel acide aminé,
et dans lequel, ladite première séquence peptidique est répétée n fois et ladite seconde séquence peptidique est répétée m fois, n et m étant des nombres entiers compris entre 1 et 5.

2. Utilisation selon la revendication 1, **caractérisée en ce que** la composition pharmaceutique fabriquée est destinée à traiter et/ou prévenir une infection fongique causée par un champignon filamenteux ou une levure.

3. Utilisation selon la revendication 1 ou 2, **caractérisée en ce que** le nombre de répétition (n) de la première séquence dudit peptide est un nombre entier compris entre 1 et 3.

4. Utilisation selon l'une quelconque des revendications 1 à 3, **caractérisée en ce que** le nombre de répétition (m) de la seconde séquence peptidique (B) dudit peptide est supérieur à 1 et **en ce que** les secondes séquences peptidiques (B) sont identiques ou différentes.

5. Utilisation selon la revendication 4, **caractérisée en ce que** les motifs peptidiques de formule βxxβ sont identiques ou différents, dans chacune des secondes séquences peptidiques (B).

6. Utilisation selon l'une quelconque des revendications précédentes, **caractérisée en ce que** les première(s) et seconde(s) séquences peptidiques dudit peptides sont:
- alternées, ou
- regroupées à l'une et/ou l'autre des extrémités N et C terminales d'une ou desdites première(s) ou seconde(s) séquences peptidiques.

7. Utilisation selon l'une quelconque des revendications précédentes, **caractérisée en ce que** la ou les premières séquences peptidiques sont regroupées à l'une et/ou à l'autre extrémités N et C terminale de la ou des secondes séquences peptidiques.

8. Utilisation selon l'une quelconque des revendications précédentes, **caractérisée en ce que** les première(s) et seconde(s) séquences peptidiques sont liées par des liaisons covalentes, avantageusement des liaisons peptidiques.

9. Utilisation selon l'une quelconque des revendications précédentes, **caractérisée en ce que** dans le motif peptidique de formule βXXβ dudit peptide, β est un acide aminé basique, de préférence choisi dans le groupe comprenant l'arginine (R) et la lysine (K), et X est choisi dans le groupe comprenant le leucine (L), la glycine (G) et l'histidine (H).

10. Utilisation selon l'une quelconque des revendications précédentes, **caractérisée en ce que** la seconde séquence peptidique de formule (B) est une séquence choisie dans le groupe comprenant SEQ ID NO.1, SEQ ID NO.2 et SEQ ID NO.3.

11. Utilisation selon l'une quelconque des revendications précédentes, **caractérisée en ce que** le peptide est choisi dans le groupe comprenant SEQ ID NO.6, SEQ ID NO.8 et SEQ ID NO.10.

12. Utilisation comme antifongique chez la plante d'un peptide tel que défini dans l'une quelconque des revendications 1 à 11.

13. Utilisation selon la revendication 12, **caractérisée en ce que** ledit peptide à une activité contre des champignons filamenteux et des levures.

14. Utilisation selon l'une quelconque des revendications 12 ou 13, **caractérisée en ce que** ledit peptide est codé par un polynucléotide compris dans un vecteur d'acide nucléique, ledit vecteur étant préalablement transformé dans ladite plante.

15. Cellule végétale comprenant une molécule d'acide nucléique selon la revendication 14 .

16. Une plante générée à partir d'une cellule végétale selon la revendication 15.

## Claims

1. Use for the production of a pharmaceutical formulation, intended to treat and/or prevent a fungal infection in humans or animals, of a peptide comprising or consisting of:
(i) a first peptide sequence according to the formula (KLAKKLAK), where K is the amino acid Lysine, L is the amino acid Leucine and A is the amino acid Alanine, and
(ii) a second peptide sequence according to the formula (B), where B is a positively charged pH-neutral peptide of 4 to 15 amino acids, comprising or consisting of at least one peptide unit according to the formula □xx□, where □is a basic amino acid and X is any amino acid,
and wherein said first peptide sequence is repeated n times and said second peptide sequence is repeated m times, n and m being integers between 1 and 5.

2. Use according to claim 1, **characterised in that** the pharmaceutical formulation produced is intended to treat and/or prevent a fungal infection caused by a filamentous fungus or a yeast.

3. Use according to claim 1 or 2, **characterised in that** the number of repetitions (n) of the first sequence of said peptide is an integer between 1 and 3.

4. Use according to any of claims 1 to 3, **characterised in that** the number of repetitions (m) of the second peptide sequence (B) of said peptide is greater than 1 and **in that** the second peptide sequences (B) are identical or different.

5. Use according to claim 4, **characterised in that** the peptide units according to the formula □xx□are identica 1 or different, in each of the second peptide sequences (B).

6. Use according to any of the above claims, **characterised in that** first and second peptide sequences of said peptide are:
- alternated, or
- grouped together at one and/or the other of the N and C terminal ends of one or said first or second peptide sequences.

7. Use according to any of the above claims, **characterised in that** the first peptides sequence(s) is/are grouped together at one and/or the other of the N and C terminal ends of the second peptide sequence(s)

8. Use according to any of the above claims, **characterised in that** the first and second peptide sequences are bound by covalent bonds, advantageously peptide bonds.

9. Use according to any of the above claims, **characterised in that** in the peptide unit according to the formula □xx□, □is a basic amino acid, preferentially selected in the group comprising arginine (R) and lysine (K), and X is selected in the group comprising leucine (L), glycine (G) and histidine (H).

10. Use according to any of the above claims, **characterised in that** the second peptide sequence according to formula (B) is a sequence selected in the group comprising SEQ ID NO.1, SEQ ID NO.2 and SEQ ID NO. 3.

11. Use according to any of the above claims, **characterised in that** the peptide is selected in the group comprising SEQ ID NO. 6, SEQ ID NO. 8 and SEQ ID NO. 10.

12. Use as an antifungal in plants of a peptide as defined in any of claims 1 to 11.

13. Use according to claim 12, **characterised in that** said peptide has an activity against filamentous fungi and yeasts.

14. Use according to any of claims 12 or 13, **characterised in that** said peptide is encoded by a polynucleotide comprised in a nucleic acid vector, said vector being previously converted in said plant.

15. Plant cell comprising a molecule of nucleic acid according to claim 14.

16. Plant generated from a plant cell according to claim 15.

## Patentansprüche

1. Verwendung zur Herstellung einer pharmazeutischen Zusammensetzung aus einem Peptid, die zur Behandlung und/oder Vorbeugung einer Pilzinfektion beim Menschen oder beim Tier bestimmt ist, umfassend oder bestehend aus:
(i) einer ersten Peptidsequenz der Formel (KLAKLAK), wobei K die Aminosäure Lysin, L die Aminosäure Leucin und A die Aminosäure Alanin ist, und
(ii) einer zweiten Peptidsequenz der Formel (B), wobei B ein Peptid aus 4 bis 15 Aminosäuren ist, das positiv bis pH-neutral geladen ist, umfassend oder bestehend aus mindestens einem Peptidmotiv der Formel □XX□, wobei □ eine basische Aminosäure und X irgend eine Aminosäure ist,
und in welcher die besagte erste Peptidsequenz n-Mal wiederholt wird und die besagte zweite Peptidsequenz m-Mal wiederholt wird, wobei n und m ganze Zahlen von 1 bis 5 sind.

2. Verwendung nach Anspruch 1, **dadurch gekennzeichnet, dass** die hergestellte pharmazeutische Zusammensetzung zur Behandlung und/oder Vorbeugung einer Pilzinfektion bestimmt ist, die von einem Fadenpilz oder einer Hefe verursacht wird.

3. Verwendung nach Anspruch 1 oder 2, **dadurch gekennzeichnet, dass** die Anzahl der Wiederholungen (n) der ersten Sequenz des besagten Peptids eine ganze Zahl von 1 bis 3 ist.

4. Verwendung nach einem der Ansprüche 1 bis 3, **dadurch gekennzeichnet, dass** die Anzahl der Wiederholungen (m) der zweiten Peptidsequenz (b) des besagten Peptids größer als 1 ist und **dadurch**, dass die zweiten Peptidsequenzen (B) identisch oder unterschiedlich sind.

5. Verwendung nach Anspruch 4, **dadurch gekennzeichnet, dass** die Peptidmotive der Formel □XX□ in allen zweiten Peptidsequenzen (B) identisch oder unterschiedlich sind.

6. Verwendung nach einem der vorangehenden Ansprüche, **dadurch gekennzeichnet, dass** die erste(n) und zweite(n) Peptidsequenz(en) des besagten Peptids:
- abwechselnd, oder
- an dem einen und/oder anderen N- und C-terminalen Ende einer oder der besagten ersten oder zweiten Peptidsequenz(en) gruppiert ist/sind.

7. Verwendung nach einem der vorangehenden Ansprüche, **dadurch gekennzeichnet, dass** die erste oder die ersten Peptidsequenz(en) an dem einen und/oder an dem anderen N- und C-terminalen Ende der zweiten Peptidsequenz(en) gruppiert ist/sind.

8. Verwendung nach einem der vorangehenden Ansprüche, **dadurch gekennzeichnet, dass** die erste(n) und zweite(n) Peptidsequenz(en) durch kovalente Verbindungen verbunden sind, vorzugsweise Peptidverbindungen.

9. Verwendung nach einem der vorangehenden Ansprüche, **dadurch gekennzeichnet, dass** im Peptidmotiv der Formel □XX□des besagten Peptids □ eine basische Aminosäure ist, vorzugsweise ausgewählt aus der Gruppe, die Arginin (R) und Lysin (K) umfasst, und X aus der Gruppe ausgewählt ist, die Leucin (L), Glycin (G) und Histidin (H) umfasst.

10. Verwendung nach einem der vorangehenden Ansprüche, **dadurch gekennzeichnet, dass** die zweite Peptidsequenz der Formel (b) eine Sequenz ist, die aus der Gruppe ausgewählt ist, die SEQ ID Nr. 1 und SEQ ID Nr. 3 umfasst.

11. Verwendung nach einem der vorangehenden Ansprüche, **dadurch gekennzeichnet, dass** das Peptid aus der Gruppe ausgewählt ist, die SEQ ID Nr. 6, SEQ ID Nr. 8 und SEQ ID Nr. 10 umfasst.

12. Verwendung eines Peptids, so wie in einem der Ansprüche 1 bis 11 definiert, als Fungizid bei der Pflanze.

13. Verwendung nach Anspruch 12, **dadurch gekennzeichnet, dass** das besagte Peptid eine Aktivität gegen Fadenpilze und Hefen hat.

14. Verwendung nach einem der Ansprüche 12 oder 13, **dadurch gekennzeichnet, dass** das besagte Peptid von einem Polynukleotid codiert ist, das in einem Nukleinsäurevektor enthalten ist, wobei der besagte Vektor zuvor in der besagten Pflanze transformiert wurde.

15. Pflanzenzelle, ein Nukleinsäuremolekül nach Anspruch 14 umfassend.

16. Eine aus einer Pflanzenzelle nach Anspruch 15 generierte Pflanze.
